# EUROPEAN PATENT APPLICATION

(11) **EP 2 357 471 A1**
(43) Date of publication of application: **17.08.2011**
(21) Application number: 09824878.4
(22) Date of filing: 09.11.2009
(51) Int. Cl.: G01N 33/48

(54) **BLOOD SERUM OR BLOOD PLASMA SEPARATION METHOD**

(30) Priority: 07.11.2008 JP 2008286435
(71) Applicant: Hitachi Chemical Company, Ltd., Tokyo 163-0449 (JP)
(72) Inventor: SUTO, Kunihiro, Hitachi-shi Ibaraki 317-8555 (JP); KOJIMA, Yasushi, Tsukuba-shi Ibaraki 300-4247 (JP); MAEKAWA, Baku, Tokyo 163-0449 (JP)
(74) Representative: HOFFMANN EITLE
(86) International application number: PCT/JP2009/069062
(87) International publication number: WO 2010/053181

(57) **Abstract**

The present invention relates to a method of separating serum or plasma which includes the steps of disposing a serum or plasma separating material in a blood collection tube; collecting blood in the collection tube; and subjecting the collection tube filled with the blood to centrifugal separation, wherein the serum or plasma separating material includes a curing component, and curing of the curing component is initiated by the centrifugal separation. According to the present invention, there is provided a method of separating serum or plasma in which upon separating the serum or plasma in a collection tube, a serum or plasma separating material is allowed to be present therein in a stable state before centrifugal separation even when contacted with blood, and in a cured state between the serum or the like and a cell-containing component after the centrifugal separation; the serum or the like and the cell-containing component can be stored in a separated state in the collection tube with a good storage stability for a long period of time, and further are excellent in stability upon freezing or thawing and upon handling of the sample; and the separating material can be cured without need of irradiation with ultraviolet ray or the like.

## Description

### TECHNICAL FIELD

The present invention relates to a method of separating serum or plasma in which a whole blood sample is separated into serum or plasma and a blood cell-containing component by a centrifugal separation procedure.

### BACKGROUND ART

In inspection or examination for blood components in clinical tests, it is required to separate whole blood into serum or plasma (hereinafter occasionally referred to as "serum or the like") and a component containing blood cells (hereinafter referred to as a "cell-containing component"). As one of the separation methods, there is known the method in which a whole blood sample is collected in a blood collection tube (hereinafter referred to merely as a "collection tube") in which a material having a specific gravity that is intermediate between those of the serum or the like and the cell-containing component is previously received, and then the thus filled collection tube is subjected to centrifugal separation to dispose the material at a position between the serum or the like and the cell-containing components to thereby separate both the blood components from each other. According to the above method, the serum or plasma thus separated can be dispensed using a pipette or by decantation without inclusion of the cell containing component in the serum or the like.
Hitherto, such a serum or plasma separating material is mainly formed of a gel-like material. For example, there has been proposed a serum separating material which contains an α-olefin-maleic acid diester copolymer having a specific range of a viscosity as a main component and whose specific gravity is adjusted to the range of 1.035 to 1.055 (refer to Patent Document 1).

However, when separating the serum or plasma using such a soft gel-like separating material, the serum or the like thus separated in an inspection site tends to subsequently suffer from re-mixing with a cell-containing component owing to vibration upon handling the sample or erroneous absorption of the separating material therein upon dispensing, which will result in failure to obtain correct inspection results. In addition, the gel-like separating material tends to cause inclusion of electrolyte components contained in the blood cells, etc., into the serum or the like through an interface between an inner wall surface of the collection tube and the separating material or through a clearance formed inside of the separating material when stored for a long period of time or when preserved in a frozen state, which also leads to erroneous measurement results.
Further, in the case that the place where blood is collected is different or remote from the place where the collected blood is inspected or examined, it might take a long time to transfer the blood sample from the blood collection site to the blood inspection or examination site. In such a case, it is not possible to use a material which is reactive with water, etc., in the blood as the serum or plasma separating material.

To solve the above conventional problems, there has been proposed the method in which after separating the serum or the like, the separating material is cured by irradiation with ultraviolet ray, etc., to completely separate respective blood components from each other (refer to Patent Documents 2 to 5).
In this method, since the separating material is completely cured by the irradiation with ultraviolet ray, the serum or the like is prevented from being re-mixed with the cell-containing component after being cured, In addition, before irradiated with ultraviolet ray, the separating material is stably present even when contacted with the blood. Therefore, even when it takes a long time from the blood collection to centrifugal separation of the blood, there will occur no significant problems.
However, it is considered that curing of the separating material by irradiation with ultraviolet ray might give any adverse influence on measurement of components whose quality tends to be deteriorated by the ultraviolet ray irradiated (for example, bilirubin). In addition, although it is usually required to sterilize a collection tube by irradiation with γ-ray, etc., the separating material disposed in the collection tube tends to be undesirably cured by the irradiation with γ-ray, etc. Therefore, there tends to arise such a problem that the collection tube is incapable of being subjected to sterilization procedure.
On the other hand, in order to avoid undesirable change in quality of the respective components by irradiation with ultraviolet ray, there is known a method of curing the separating material by irradiating a reduced amount of ultraviolet ray thereto. However, since the respective blood components, are present on both upper and lower sides of the separating material, the ultraviolet ray irradiated fails to reach a central portion of a resin of the separating material. Thus, it will be difficult to completely cure the resin inclusive of an inside portion thereof in the collection tube. As a result, there also tends to occur such a problem that the cell-containing component is re-mixed in the serum or the like, similarly to the above case where the uncured gel is used as the separating material.

Also, there has been proposed a tubular blood separating tube in which a water-repellent surface is deposited on an inner wall surface thereof at a position where a blood separating material is anchored or stopped after subjected to a centrifugal separation procedure (refer to Patent Document 6). In Patent Document 6, it is described that a separating layer formed in the blood separating tube has an increased adhesion, to the inner wall surface of the blood collection tube, so that even when taking out serum therefrom after a long time has elapsed from the centrifugal separation procedure, it is possible to dispense pure serum. In addition, the serum separating material used in Patent Document 6 might be similar to that of the present invention in such a point that the specific gravity thereof is adjusted to 1.04, and the serum is separated by the centrifugal separation method. However, the serum separating material used in Patent Document 6 has such a problem that after stored for a long period of time or after preserved in a frozen state, it is not possible to prevent a part of a blood cell component from being mixed in the serum component through an interface between an inner wall surface of the blood collection tube and the separating material or through a clearance formed within the separating material.
Further, there has been proposed a serum or plasma separating material including a serum or plasma separating composition simply received in a container according to requirements, in which the receiving member has a weakly sealed portion which is broken by centrifugal separation to allow the gel-like composition to leak out therefrom (refer to Patent Document 7). However, the separating composition described in Patent Document 7 is in the form of a gel-like material, and therefore has the above-mentioned problems. In addition, it will be difficult to adjust a sealing strength of the weakly sealed portion. When the sealing strength is too low, the composition tends to be readily leaked out upon handling, whereas when the sealing strength is too high, the weakly sealed portion tends to be hardly broken upon the centrifugal separation.

Also, Patent Document 8 discloses a blood separating filter for separating whole blood into a blood cell component and plasma or serum (refer to claims and paragraph [0001] of Patent Document 8), and further describes that synthetic polymer fibers, glass fibers and porous polymers are used as a material of the fiber (refer to paragraph [0024] of Patent Document 8). However, Patent Document 8 relates to a method using no centrifugal separation (refer to paragraph [0003] of Patent Document 8), and does not aim at solving the problems described in the present invention and is therefore quite different in inventive concept from that of the present invention.
In addition, there has been proposed a blood tester which includes an outer tube in the form of a closed-end tubular container having an opening at one bottom end, an inner tube secured to an inner wall surface of the outer tube to form a telescopic structure which is out of contact with the outer tube, a serum separating material slidably moved on an inner wall surface of the inner tube, and a hermetically sealing plug member, in which the serum separating material has a specific gravity of 1.03 or more and preferably 1.05 or more (refer to claims and paragraphs [0020] and [0021] of Patent Document 9). In Patent Document 9, it is described that the container used therein is capable of subjecting the separated blood components to freeze-drying without need of transferring serum to another container (refer to paragraphs [0010] to [0012] of Patent Document 9). However, in the method described in Patent Document 9, although the blood components are suitably stored owing to an elasticity of the inner tube, it is considered that the elasticity of the inner tube is deteriorated by creep phenomenon during a long-term storage. Therefore, the method described in Patent Document 9 also has such a problem that after preserved for a long period of time or after stored in frozen state, it is not possible to prevent a part of the cell-containing component from being mixed with the serum or the like through an interface between an inner wall surface of a blood collection tube and the separating material or through a clearance formed within the separating material.

Patent Document 1: Japanese Patent Publication No. S63-48310
Patent Document 2: US. Patent No. 6248844
Patent Document 3: US. Patent Application Laid-Open No. 2007/187341
Patent Document 4: US. Patent Application Laid-Open No. 2008/108493
Patent Document 5: US. Patent Application Laid-Open No. 2008/132874
Patent Document 6: Japanese Patent Publication No. H6-77014
Patent Document 7: Japanese Patent Application Laid-Open No. 2001-318091
Patent Document 8: Japanese Patent Application Laid-Open No. 2006-3340
Patent Document 9: Japanese Patent Application Laid-Open No. H9-222427

### DISCLOSURE OF THE INVENTION

An object of the present invention is to provide a method of separating serum or plasma in which upon separating the serum or plasma in a collection tube, a separating material is allowed to be present therein in a stable state before centrifugal separation even when contacted with blood, and in a cured state between the serum or the like and a cell-containing component after the centrifugal separation; the serum or the like and the cell-containing component can be stored in a separated state with a good storage stability in the collection tube for a long period of time and further are excellent in stability upon freezing or thawing and upon handling of the sample; and the separating material can be cured without need of irradiation with ultraviolet ray or the like.

As a result of intense and extensive researches, the present inventors have found that the above conventional problems can be solved by incorporating a curing component in the separating material and initiating curing of the curing component by a centrifugal separation procedure. The present invention has been accomplished on the basis of the finding, This, the present invention relates to a method of separating serum or plasma which includes the steps of disposing a serum or plasma separating material in a blood collection tube; collecting blood in the collection tube; and subjecting the collection tube filled with the blood to centrifugal separation, wherein the serum or plasma separating material includes a curing component, and curing of the curing component is initiated by the centrifugal separation.

According to the method of the present invention upon conducting the separation procedure in a collection tube, before centrifugal separation, the serum or plasma separating material used therein is allowed to be present in a stable state even when contacted with blood so that occurrence of any inconveniences can be avoided even when a long time is required from collection or the blood up to inspection or examination thereof, whereas, after the centrifugal separation, the serum or the like and a cell-containing component which are separated from each other can be stored in a separated state with a good storage stability for a long period of time and further are excellent in stability upon freezing or thawing and upon handling of the sample. In addition, according to the method of the present invention, the serum or plasma separating material can be cured without need of irradiation with ultraviolet ray, so that a blood test can be carried out without taking into consideration adverse influence of the ultraviolet ray, and a sterilization procedure by irradiation with γ-ray can be carried out without any inconveniences.

### BRIEF DESCRIPTION OF THE DRAWINGS

FIGS. 1-1 to 1-3 are schematic views showing one embodiment of a method according to the present invention.
FIGS. 2-1 to 2-3 are schematic views showing another embodiment of the method according to the present invention.
FIGS. 3-1 to 3-3 are schematic views showing a further embodiment of the method according to the present invention.
FIGS. 4-1 to 4-3 are schematic views showing the other embodiment of the method according to the present invention.
FIGS. 5-1 to 5-3 are schematic views showing the still other embodiment of the method according to the present invention.
FIGS. 6-1 to 6-3 are schematic views showing the still other embodiment of the method according to the present invention.
FIGS, 7-1 to 7-3 are schematic views showing the still other embodiment of the method according to the present invention.
FIGS. 8-1 to 8-3 are schematic views showing the still other embodiment of the method according to the present invention.
FIGS. 9-1 to 9-3 are schematic views showing the still other embodiment of the method according to the present invention.
FIGS. 10-1 to 10-3 are schematic views showing the still other embodiment of the method according to the present invention.

### Explanation of Reference Numerals

1: Blood collection tube; 2: Tube cavity; 3: Lid; 4: Moisture curing component; 4': Two-part liquid curing type component; 5: Blood isolating material; 6: Whole blood; 7: Serum or plasma (serum or the like); 8: Blood cell-containing component; 9: Capsule; 10: High-specific gravity solid; 11: Container; 12: Lid; 21: A liquid (one components of the two-part liquid curing type component); 22: B liquid (the other component of the two-part liquid curing type component); 23: Film; 31: Groove or hole; 41: Surface-treated beads; 42: Water-soluble resin; 51: Molded article

### BEST MODE FOR CARRYING OUT THE INVENTION

The method of separating serum or plasma according to the present invention includes the steps of disposing a serum or plasma separating material (hereinafter occasionally referred to merely as a "separating material,") in a blood collection tube; collecting blood in the collection tube; and subjecting the collection tube filled with the blood to centrifugal separation, wherein the serum or plasma separating material includes a curing component, and curing of the curing component is initiated by the centrifugal separation.
More specifically, in order to initiate curing of the separating material by the centrifugal separation, the curing rate of the curing component in the separating material as well as the time period from collection of blood to initiation of the centrifugal separation may be suitably controlled. More preferably, the curing component is isolated from factors capable of initiating a curing reaction thereof such that the isolating condition is released by the centrifugal separation.

In the method of the present invention, the serum or the like and the cell-containing component are separated from each other by the centrifugal separation. Therefore, the curing component contained in the separating material preferably has a specific gravity which is intermediate between those of the serum or the like and the cell-containing component. More specifically, the curing component contained in the separating material preferably has a specific gravity of from 1.03 to 1.09, more preferably from 1.03 to 1.07 and still more preferably from 1,035 to 1.055. In order to control the specific gravity of the curing component contained in the separating material to the above specified range, the kind of a resin or compound used as the curing component as well as the kinds of monomers used for forming the resin or compound, etc., may be appropriately selected. The specific gravity of the curing component is preferably controlled in the above manner from the viewpoint of a good stability of the resulting separating material. On the other hand, there may also be used an alternative method in which the specific gravity of the curling component is adjusted to the above specified range by compounding a specific gravity modifier therein. This method is advantageous in that the specific gravity is relatively easily controlled.
Specific examples of the specific gravity modifier include silica and zeolite such as "Aerosil 130", "Aerosil R972" and "Aerosil QX50" all available from Nippon Aerosil Co., Ltd.; bentonites such as "Bentone 38" and "Bentone SD-1" both available from Elements Specialties Corp.; minerals such as smectite clay, kaolin clay and antigorite clay; inorganic fine particles containing calcium carbonate, titanium dioxide or the like; and polymer fine particles of polystyrenes, polyurethanes, polymethyl (meth)acrylates, acrylonitrile-styrene copolymers and rubbers, These specific gravity modifiers may also be used as a viscosity modified. In addition, the inorganic fine particles may also be used as a thixotropy imparting agent,
In the case where the below-mentioned moisture curing resin is used as the curing component, if only the specific gravity modifier is added to the moisture curing resin, the viscosity of the resulting moisture curing resin composition, is preferable from 0.1 to 1000 Pa·s, preferably from 0.5 to 500 Pa·s, and more preferably from 1 to 100 Pa·s. When the viscosity of the resin composition is 0.1 Pa·s or more, the specific gravity modifier and the resin can be suitably prevented from being separated from each other when subjected to centrifugal separation. On the other hand, when the viscosity of the resin composition is 1000 Pa·s or less, the resin composition has an adequate viscosity without becoming excessively high, so that the separating material can exhibit a sufficient adhesion to a wall surface of the collection tube without deterioration in bonding property therebetween when subjected to the centrifugal separation.

In the method of the present invention, the curing component contained in the separating material is not particularly limited, and any suitable materials may be used therefor as long as they can be cured by any factors. Examples of the curing component may include monomers or oligomers which can be cured by polymerization, polymers which can be cured by crosslinking, and surface treated particles which are capable of being cured by forming a crosslinked product when contacted with each other. In the followings, the curing component contained in the separating material is explained for each kind thereof.

### (1) When using a moisture curing component:

When using a moisture curing component as the curing component, the serum or plasma separating material initiates a curing reaction thereof by the action of water contained in blood. Therefore, before separating the serum or the like and the cell-containing component from each other by centrifugal separation, the separating material is prevented from coming into contact with water in the blood. In order to prevent the contact between the separating material and the blood, a blood isolating material is preferably disposed so as to prevent the moisture curing component from coming into contact with the blood. For example, for this purpose, there may be used the method in which the moisture curing component is enclosed in a capsule, the method in which the moisture curing component is received in a container, the method in which a isolating wall such as a filter is disposed between the separating material and the blood.

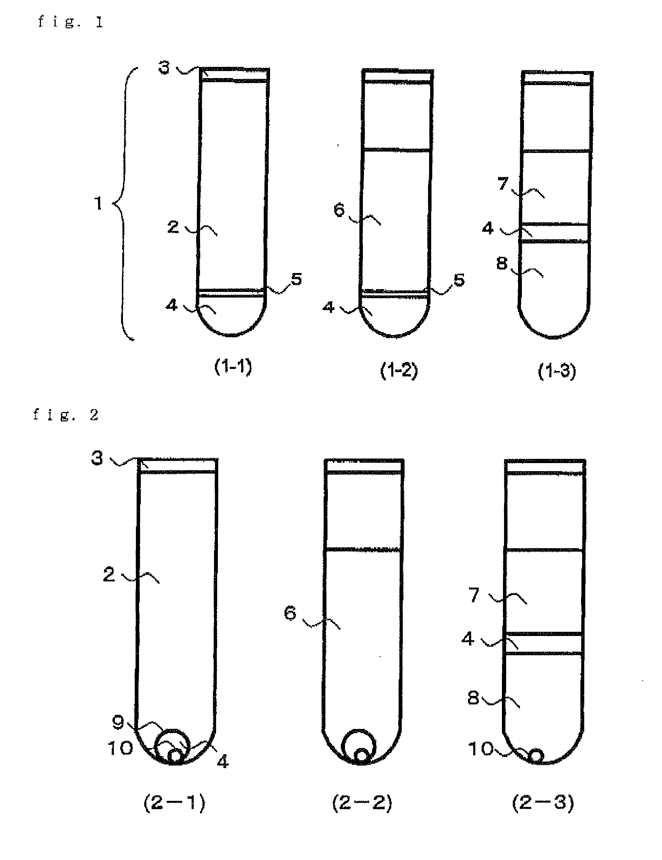

FIGS. 1-1 to 1-3 are schematic views showing one embodiment of the method according to the present invention in which an isolating wall is provided.

FIG. 1-1 shows the collection tube 1 in which the moisture curing component 4 is disposed at a bottom of the collection tube 1. In the embodiment shown in FIGS. 1-1 to 1-3, the blood isolating material 5 is disposed on a surface of the moisture curing component in order to prevent the moisture curing component from coming into contact with the blood. The blood isolating material 5 might not be necessarily requited depending upon the curing rate of the moisture curing component 4 and the time period taken from collection of the blood to initiation of the centrifugal separation. However, the embodiment using the blood isolating material 5 is preferable because the moisture curing component 4 and water contained in the blood can be completely separated from each other, and curing of the moisture curing component 4 can be initiated by the centrifugal separation.

FIG. 1-2 shows the condition immediately after whole blood 6 is collected in the collection tube 1. In this condition, the moisture curing component and the blood are prevented from contacting with each other by the blood isolating material 5 and therefore no curing of the moisture curing component is initiated.

As the method of bringing the moisture curing component and blood into contact with each other by centrifugal separation, there may be used the method in which the blood isolating material is disposed such that bonding between the blood isolating material and an inner wall surface of the collection tube is released by a gravity owing to the centrifugal separation, or the method in which a solid having a high specific gravity (hereinafter referred to as a "high-specific gravity solid") is disposed in the vicinity of the blood isolating material. In the former method, the material of the blood isolating material may be selected from those materials which are capable of releasing a bonding force of a material which serves for bonding the blood isolating material to an inner wall surface of the collection tube (such as the blood isolating material itself, a bonding agent, an adhesive and a tackifier). In the latter method, before the centrifugal separation, the moisture curing component and blood are still prevented from coming into contact with each other by the blood isolating material so that no curing reaction of the moisture curing component proceeds, and upon the centrifugal separation, the high-specific gravity solid serves for breaking the blood isolating material so that the moisture curing component and blood are brought into contact with each other to thereby initiate curing of the moisture curing component. The high-specific gravity solid may be disposed in various locations or configurations. The high-specific gravity solid may be disposed above the blood isolating material as described in detail hereinafter. Also, the latter method in which the high-specific gravity solid may be disposed in the vicinity of a capsule as the blood isolating material includes such a technical concept that the high-specific gravity solid is enclosed in the capsule together with the moisture curing component.

Upon selecting materials, sizes, thicknesses, masses, etc., of the blood isolating material and the high-specific gravity solid, it should be noted that before the centrifugal separation, the moisture curing component and blood are prevented from coming into contact with each other by the blood isolating material, and after the centrifugal separation, the blood isolating material is at least partially readily broken by the high-specific gravity solid to allow the moisture curing component and blood to contact with each other. In addition, the blood isolating material and the high-specific gravity solid are each preferably made of a material having a higher specific gravity than that of the moisture curing component such that both the materials are allowed to be present in the cell-containing component after completion of the centrifugal separation. This is because the cell-containing component is usually excluded from objective components to be examined in blood tests, and there therefore occur no significant problems even though the cell-containing component contains the blood isolating material and the high-specific gravity solid.

In the embodiment shown in FIGS. 1-1 to 1-3, when being subjected to centrifugal separation, the blood isolating material 5 is displaced or broken to allow the moisture curing component and the blood to contact with each other, so that curing of the moisture curing component is initiated.
More specifically, upon the centrifugal separation, the moisture curing component and the cell-containing component are exchanged in positions with each other to thereby bring the moisture curing component into contact with the blood, so that curing of the moisture curing component is initiated. At the same time, as shown in FIG. 1-3, the blood is separated into serum or plasma 7 and the cell-containing component 8. After or while the moisture curing component 4 is moved to the position between the serum or the like 7 and the cell-containing component 8, curing of the moisture curing component proceeds, so that the upper and lower blood components are prevented from being mixed with each other.

The blood isolating material 5 is not particularly limited, and any suitable material may be used therefor as long as the moisture curing component and the blood are isolated from each other by the material. The blood isolating material 5 may be either a liquid material or a solid material. In view of a stability upon transportation and an isolating property upon collecting the blood, the blood isolating material is preferably in the form of a film. Examples of the material of the liquid blood isolating material include mineral oils, vegetable oils and silicone oils. The material of the solid blood isolating material may be the same as or different from a cured product of the moisture curing component, and may also be either an elastic material or a non-elastic material Specific examples of the suitable material of the solid, blood isolating material include films or gels formed of polyolefins such as polyethylene and polypropylene; polystyrenes; acrylates such as polymethyl methacrylate; polyesters such as polyethylene terephthalate; polyethers such as polyethylene glycol; fluororesins such as polytetrafluotoethylene; silicone resins such as poly(dimethyl siloxane); polysaccharides such as pullulan, carageenan, collages, gelatin and starches; water-soluble polymers such as proteins and polyvinyl alcohol; rubbers such as natural rubbers and urethane rubbers; and metals such as aluminum. In addition, the blood isolating materials 5 may be constituted from a single material or a plurality of materials. The blood isolating material in the form of a membrane or a film preferably has a thickness of from 1 to 10000 µm and more preferably from 5 to 500 µ m.

As the method of displacing or breaking the blood isolating material 5 to allow the moisture curing component and the blood to contact with each other, there may be used, for example, the method in which the blood isolating material 5 is prepared from a high-specific gravity solid so as to be movable by a gravity of the high-specific gravity solid upon the centrifugal separation. In addition, there may also be used an alternative method in which a high-specific gravity solid is disposed above the blood isolating material 5 so that the blood isolating material is moved or broken by the gravity of the high-specific gravity solid upon the centrifugal separation.

Next, in the embodiment shown in FIGS. 2-1 to 2-3, the moisture curing component is enclosed in a capsule 9 as the blood isolating material (refer to FIG. 2-1). In this method, as shown in FIG, 2-2, even when whole blood 6 is collected in the collection tuber the moisture curing component is free from contact with water, and therefore curing of the moisture curing component is not initiated. Thereafter, the capsule is broken by the centrifugal separation, so that the moisture curing component is brought into contact with the blood to initiate curing of the moisture curing component. As shown in FIG. 2-3, after completion of the centrifugal separation, the serum or the like 7 and the cell-containing component 8 are separated from each other, and the moisture curing component 4 becomes cured after or while being disposed therebetween, so that the upper and lower blood components can be prevented from being mixed again with each other.
As the method of breaking the capsule 9 as the blood isolating material, there may be mentioned, for example, the method in which a high-specific gravity solid 10 is also enclosed in the capsule. When being subjected to centrifugal separation under this condition, the capsule 9 is holed by a gravity of the high-specific gravity solid 10 upon the centrifugal separation, so that the moisture curing component 4 enclosed in the capsule is discharged through the resulting opening out of the capsule. In addition, the capsule may have a reduced thickness. In such a case, the capsule can be broken only by the centrifugal separation procedure without using the high-specific gravity solid.

As the high-specific gravity solid 10, there may be used plastic materials, ceramic materials such as silica and alumina, and metals. The specific gravity of the high-specific gravity solid is preferable in the range of from 1.1 to 15.0, more preferably from 1.2 to 10.0 and especially preferably from 1.3 to 8.0.
Meanwhile, in the above embodiment, the high-specific gravity solid is enclosed in the capsule. However, it is not necessarily required that the high-specific gravity solid is enclosed in the capsule. The high-specific gravity solid may be disposed outside the capsule in the vicinity thereof such that the capsule is broken from outside to discharge the moisture curing component out of the capsule. In addition, the high-specific gravity solid may be formed integrally with the blood isolating material 5. For example, the high-specific gravity solid may be disposed on a surface of a film as the blood isolating material 5. In this case, the blood isolating material is preferably formed of a material having a less elongation and an adequate strength.
The high-specific gravity solid may have various shapes including a spherical shape, a polyhedral shape, a cylindrical shape, a rectangular parallelopiped shape and a plate shape. Among these shapes, preferred are those shapes which are chamfered so as to hardly undergo physical breakage upon transportation, and more preferred is a spherical shape. The single high-specific gravity solid may be used, or a plurality of the high-specific gravity solids may also be used.
The size of the high-specific gravity solid is not particularly limited, and the high-specific gravity solid may have any diameter as long as it can be received in the collection tube. More specifically, the high-specific gravity solid preferably has a diameter smaller by 1 mm or more than a diameter of the collection tube so as not to inhibit movement of blood therein upon the centrifugal separation. The lower limit of the diameter of the high-specific gravity solid is not particularly limited and may be appropriately determined as long as the high-specific gravity solid has a sufficient weight capable of discharging the moisture curing component. The suitable diameter of the high-specific gravity solid is usually 0.5 mm or more since such a high-specific gravity solid has a weight which is enough to discharge the moisture curing component.

As the material of the capsule 9, there may be suitably used the same material as the solid blood isolating material used for the above blood isolating material 5. The thickness of the capsule may also be the same as that of the blood isolating material 5 having a film-like shape. In addition, the capsule may have various shapes including a spherical shape, a cylindrical shape, a polyhedral shape and a rectangular parallelopiped shape.
Meanwhile, the embodiment as shown in FIGS. 2-1 to 2-3 in which the separating material is enclosed in the capsule may be used in combination with the embodiment as shown in FIGS. 1-1 to 1-3 in which the blood isolating material 5 is used.

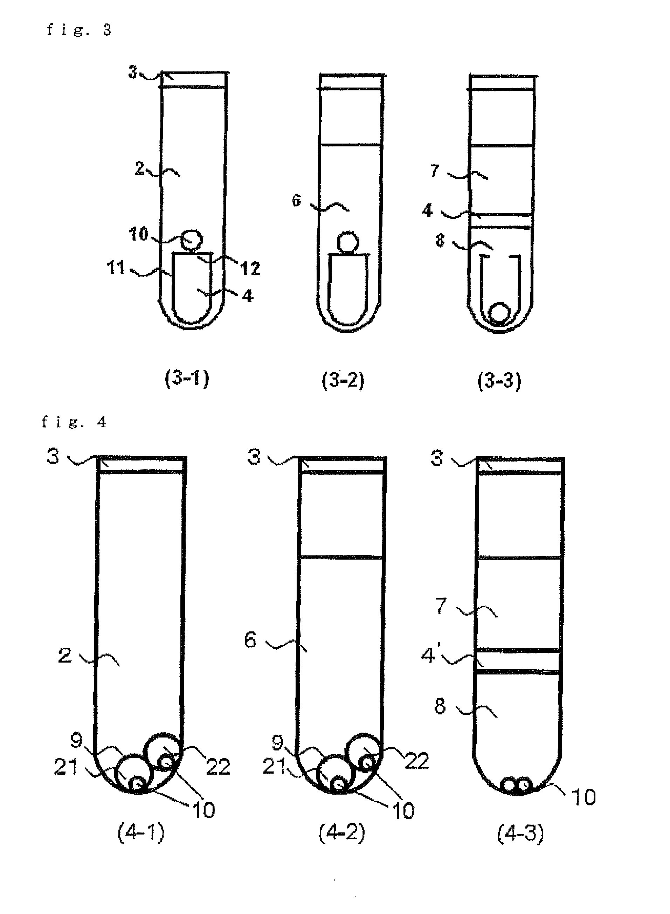

As shown in FIGS. 3-1 to 3-3, there may also be used the method in which the moisture curing component is received in a container 11, and then the container 11 is closed and sealed by a film-like lid 12 as the blood isolating material (refer to FIG. 3-1). In this method, as shown in FIG. 3-2, even when whole blood 6 is collected in the collection tube, the moisture curing component is prevented from contacting with water, so that curing of the moisture curing component is not initiated. Thereafter, the film-like lid 12 is broken upon the centrifugal separation to allow the moisture curing component to come into contact with the blood, so that curing of the moisture curing component is initiated. As shown in FIG. 3-3, after completion of the centrifugal separation, the serum or the like 7 and the cell-containing component 8 are separated from each other, and the moisture curing component 4 becomes cured after or while being disposed therebetween, so that the upper and lower blood components can be prevented from being mixed with each other.
The container 11 used above is a blood isolating material which may be constituted from a molded article or a film, and the lid 12 may also be constituted from a film, etc. The container may be provided with one or more openings. When using the container having one opening, a plastic molded container such as, for example, a press-through-package (PTP) may be used as the blood isolating material, and is filled with the moisture curing component, and then closed with a lid as the blood isolating material such as an aluminum vapor deposited film and an aluminum foil. When using the container having two opening, a film is attached to a lower opening portion of a tubular container, and after filling the moisture curing component therein, an upper opening portion, of the container is closed with a lid. In such a case, since the container is holed at its upper and lower portions by the high-specific gravity solid, the moisture curing component can be more readily discharged out of the capsule. The high-specific gravity solid 10 may be disposed outside the container 11. In this case, the film-like lid is broken by the high-specific gravity solid 10 when subjected to centrifugal separation, so that the moisture curing component can be discharged out of the container 11. At this time, in order to facilitate breakage of the film-like lid by the high-specific gravity solid, the high-specific gravity solid may be bonded to an upper surface of the film-like lid (FIG. 3-2).

The film used as the lid 12 is preferably made of a material capable of fully sealing the moisture curing component 4 therein in an ordinary condition and capable of being readily broken by the high-specific gravity solid 10 when subjected to the centrifugal separation. More specifically, the film as the lid 12 preferably has a burst strength of from 1 to 10000 kPa (as measured according to JIS P8112) and a breaking elongation of from 1 to 40%. The film having a burst strength of 1 kPa or more is free from brittleness and can exhibit a sufficient sealing property. On the other hand, the film having a burst strength of 10000 kPa or less can be suitably broken by the high-specific gravity solid 10 when subjected to the centrifugal separation. From the above viewpoints, the burst strength of the film as the lid 12 is more preferably from 5 to 1000 kPa and especially preferably from 10 to 500 kPa in order to further enhance the sealing property and ensure breakage thereof.
In addition, the film having a breaking elongation of 1% or more (as measures according to JIS P8113) is free from brittleness and can exhibit a sufficient sealing property. On the other hand, the film having a breaking elongation of 40% or less can be suitably broken by the high-specific gravity solid 10 when subjected to the centrifugal separation. From the above viewpoints, the breaking elongation of the film as the lid 12 is more preferably from 5 to 35% and especially preferably from 10 to 30% in order to further enhance the sealing property and ensure breakage thereof.
The film may be formed from a single polymer or a plurality of polymers and additives such as a filler, and the above burst strength and breaking elongation of the film may be suitably controlled by using adequate combination of these components or suitably adjusting contents thereof, etc.

The extent of curing of the separating material when the moisture curing component is used therein may be determined such that no breakage of a cured surface of the moisture curing component occurs owing to vibration and lay-down upon handling or contact with a pipette after completion of the centrifugal separation. The curing time of the moisture curing component may be optionally determined. In addition, it is preferred that curing of the moisture curing component be completed upon termination of the centrifugal separation. It is more preferred that the curing of the moisture curing component be completed during the centrifugal separation procedure after separating whole blood into serum or plasma and the cell-containing component by the moisture curing component.
Further, the high-specific gravity solid 10 is allowed to be present in the cell-containing component 8 after completion of the centrifugal separation as shown in FIGS. 2-3 and 3-3, and the container 11 is also allowed to be present in the cell-containing component 8 after completion of the centrifugal separation as shown in FIG. 3-3. However, even in such a case, there occur no significant problems because the cell-containing component is usually excluded from objective components to be examined in blood tests.

### (2) When using a two-part liquid curing type component:

Next, the method using a component of a two-part liquid curing type as the curing component contained in the separating material (hereinafter referred to merely as a "two-part liquid curing type component") is explained. When using the two-part liquid cubing type component, it is important that two liquids (referred to as an "A liquids" and a "B liquid", respectively) are prevented from contacting with each other, and brought into contact with each other by centrifugal separation.

More specifically, the embodiment shown in FIGS. 4-1 to 4-3 is illustrated. That is, there is used the method in which the two liquids of the two-part liquid curing type component are enclosed in respective capsules to prevent contact therebetween. For example, as shown in FIG. 4-1, the A liquid 21 and the B liquid 22 are enclosed in respective capsules 9 and therefore prevented from contacting with each other, so that curing of the two-part liquid curing type component is not initiated. Under this condition, even when whole blood is collected in a collection tube, the capsules 9 are not broken, so that the A liquid 21 and the B liquid 22 are prevented from contacting with each other (refer to FIG. 4-2). Next, when subjected to centrifugal separation, high-specific gravity solids 10 disposed within the respective capsules 9 break the capsules owing to their gravity upon the centrifugal separation so that both the liquids are mixed with each other. Namely, the A and B liquids are contacted with each other upon the centrifugal separation so that curing of the two-part liquid curing type component is initiated. As shown in FIG. 4-3, after completion of the centrifugal separation, the serum or the like 7 and the cell-containing component 8 are separated from each other, and the two-part liquid curing type component 4' is cured therebetween so that the upper and lower blood components are prevented from being mixed with each other. Meanwhile, there may also be used the method in which the high-specific gravity solids are disposed outside the respective capsules such that the capsules are broken by the high-specific gravity solids upon the centrifugal separation. Further, there may also be used the method in which the capsules having a reduced thickness are broken only by centrifugal separation without using the high-specific gravity solids.

The degree of curing of the separating material using the two-part liquid curing type component is preferably adjusted such that when the two liquids contained therein are mixed with each other during the centrifugal separation procedure to separate whole blood into serum or plasma and the cell-containing component, the resulting cured product of the two-part liquid curing type component is free from breakage upon pipetting, etc. The two-part liquid curing type component is more preferably completely cured upon termination of the centrifugal separation.

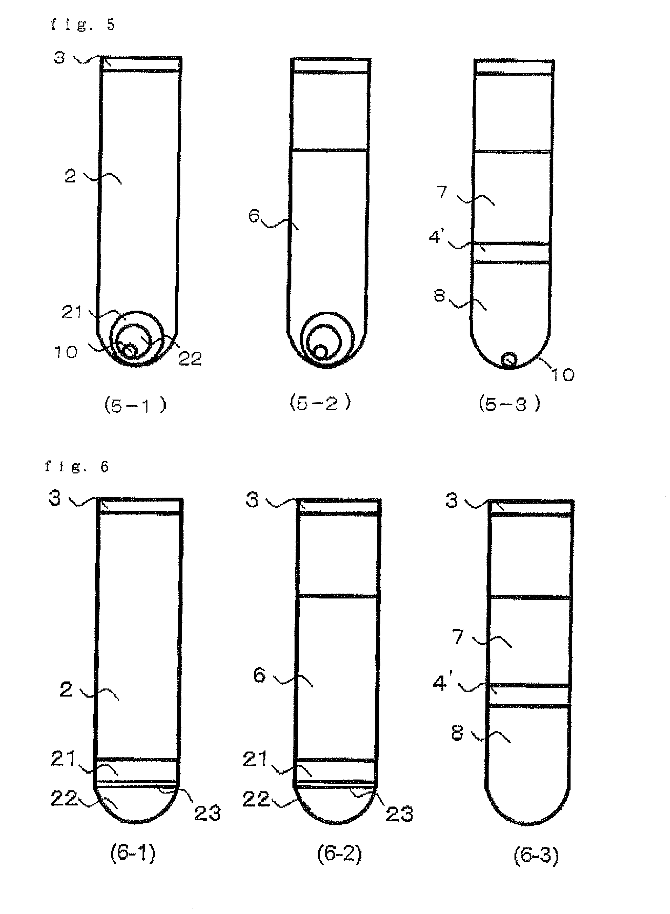

Further, as shown in FIGS. 5-1 to 5-3, one of the capsules may be enclosed within the other capsule. More specifically, in FIG. 5-1, there is shown a double capsule structure in which a capsule enclosing the B liquid 22 is enclosed in a capsule enclosing the A liquid 21, and the high-specific gravity solid 10 is received in the capsule enclosing the B liquid 22. When subjecting the double capsule structure to centrifugal separation, the capsules are broken by the high-specific gravity solid 10, so that the A and B liquids are mixed with each other to initiate curing of the two-part liquid curing type component. In addition, by reducing a thickness of the respective capsules, it is possible to break the capsules only by the centrifugal separation procedure without using the high-specific gravity solid.
Meanwhile, the respective capsules may be formed of the same material as used above.

In another embodiment using the two-part liquid curing type component, as shown in FIGS. 6-1 to 6-3, there may be used the method in which the A liquid 21 and the B liquid 22 of the two-part liquid curing type component are laminated on each other through a film 23 in a cavity of the tube. Before the centrifugal separation, the film is disposed between the two liquids to allow the two liquids to be out of contact with each other, so that curing of the two-part liquid curing type component is not initiated (refer to FIG. 6-1). Under this condition, even though whole blood is collected in the collection tube, the two liquids can be kept in the out-of-contact condition (refer to FIG. 6-2). Next, when subjected to the centrifugal separation, the film 23 is broken by a gravity upon the centrifugal separation so that the A and B liquids are brought into contact with each other to initiate curing of the two-part liquid curing type component. Further, as shown in FIG. 6-3, after completion of the centrifugal separation, the serum or the like 7 and the cell-containing component 8 are separated from each other, and the two-part liquid curing type component 4' is allowed to be present in a cured state therebetween. As a result, the upper and lower blood components can be prevented from being mixed with each other.
The film used above is not particularly limited, and any suitable film may be used as long as the A liquid 21 and the B liquid 22 are not mixed together, and the film is broken or displaced by the centrifugal separation procedure. The film may be formed of the same material as the above blood isolating material. The film preferably has a less elongation and an adequate strength.

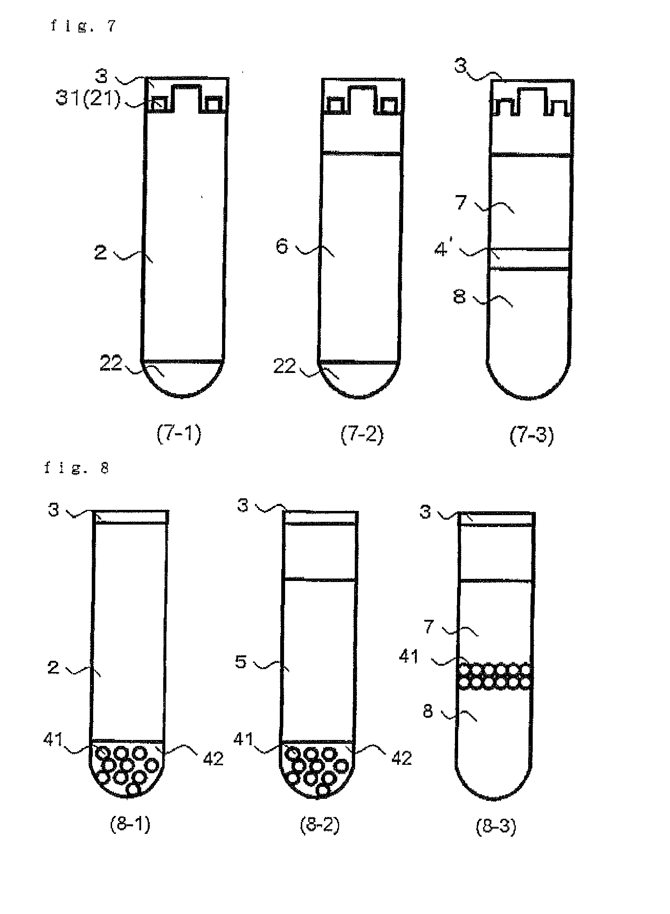

In addition, as shown in FIGS. 7-1 to 7-3, there may also be used such a configuration in which the A liquid 21 is disposed in a lid for the collection tube 1, whereas the B liquid 22 is disposed within the collection tube such that the A liquid is introduced from the lid into the collection tube upon the centrifugal separation and mixed with the B liquid. More specifically, the lid 3 is formed with a groove or hole 31 capable of receiving one of the liquids of the two-part liquid curing type component (A liquids). The A liquid 21 is filled in the groove or hole 31, and then sealed therein. Meanwhile, the method of disposing the A liquid in the lid is not particularly limited, and the groove or hole of the lid has any suitable shape as long as run-off or leakage of the A liquid from the lid is prevented from occurring upon contacting with blood collected. Examples of the shape of the groove or hole of the lid include a cylindrical tubular shape and a rectangular parallelopiped shape.
On the other hand, the B liquid 22 is disposed at a bottom of the collection tube 1. Before the centrifugal separation, the two liquids are out of contact with each other, and therefore no curing of the two-part liquid curing type component is initiated (FIG. 7-1). Under this condition, event though whole blood 6 is collected in the collection tube, the A and B liquids are still kept out of contact with each other (refer to FIG. 7-2).
When subjected to the centrifugal separation under the above condition, the A liquid 21 is flowed out of the lid into the whole blood 6 by a gravity owing to the centrifugal separation, whereas the B liquid is also mixed in the whole blood 6, so that the A and B liquids are brought into contact with each other to initiate curing of the two-part liquid curing type component. The A liquid is preferably imparted with a thixotropic property such that run-off of the A liquid from the lid is prevented upon ordinary handling procedure but the A liquid is introduced from the lid into the collection tube owing to a stress applied thereto upon the centrifugal separation.
As shown in FIG. 7-3, after completion of the centrifugal separation, the serum or the like 7 and the cell-containing component 8 are separated from each other, and the two-part liquid curing type component 4' is allowed to be present in a cured state therebetween, so that the upper and lower blood components can be prevented from being mixed with each other.

### (3) When using surface-treated beads:

Next, the method using surface-treated beads as the curing component in the separating material is explained. The surface treated beads used herein are beads which are cured by undergoing a crosslinking reaction therebetween, etc., when they are contacted with each other. As shown in FIG. 8-1, before the centrifugal separation, the surface-treated beads 41 and a water-soluble resin 42 are introduced in the form of a mixture thereof into the collection tube 1. Therefore, the surface-treated beads are kept out of contact with each other. When blood is collected in the collection tube (FIG. 8-2), the water-soluble resin is dissolved in the blood, so that the surface-treated beads are diffused in the blood. Then, when subjected to the centrifugal separation, the beads having a specific gravity intermediate between those of the serum or the like and the cell-containing component are gathered and disposed between the serum or the like and the cell-containing component and brought into contact with each other as shown in FIG. 8-3. At this time, functional groups being present on the surface of the respective beads begin to be crosslinked with each other so that a high-strength separate layer is formed.

The surface treatments of the beads may be carried out by various methods. For example, there may be used a wet method in which the surface of the respective beads is treated with a solution prepared by dissolving a resin containing a large amount of a functional group, e.g., containing a carboxyl group or a glycidyl group at a high concentration, in a solvent using a spray-coating apparatus equipped with a fluidized bed, etc., and a method in which particles obtained by pulverizing a solid resin similarly containing a large amount of a functional group, e.g., containing a carboxyl group or a glycidyl group at a high concentration, are directly deposited on the respective beads as mother particles by high-speed air flow impact method, etc. In addition, as an alternative method of surface-treating the beads, there may also be used the method in which after synthesizing core particles by suspension polymerization, a shell layer having a high function group concentration is successively formed thereon by suspension polymerization to obtain core/shell type particles.

The particle size of the surface-treated beads is not particularly limited, and the beads have any suitable particle size as long as the aimed effects of the present invention can be attained, The surface-treated beads may have a particle size of from about 1 µ m to about 10 mm.

It is also required that the water-soluble resin 42 is formed of a material having no adverse influence on blood tests even when dissolved in blood. Examples of the suitable water-soluble resin include polyvinyl alcohol, polyethylene glycol, polyvinyl pyrrolidone, water-soluble silicones, dextrin and cellulose derivatives (such as, for example, carboxymethyl cellulose, hydroxyethyl cellulose and methyl cellulose).

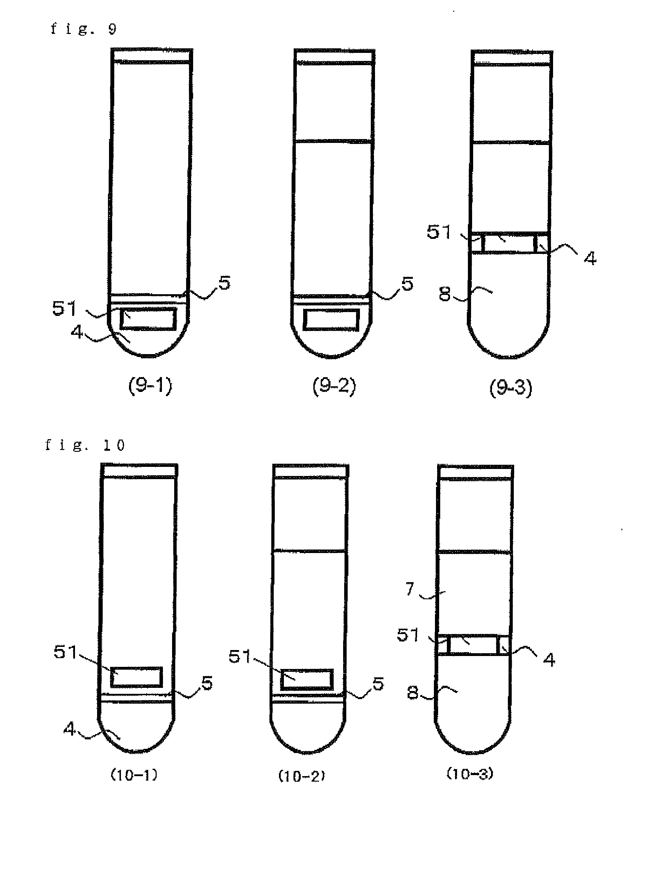

The separating material used in the present invention may also contain, if required, a reinforcing material such as beads, powders and molded articles. When the separating material contains the reinforcing materials, even the separating material having a low hardness can exhibit an increased strength. For example, in the case where the blood components are examined using an automatic analyzer in a clinical test, it is possible to prevent a probe of the automatic analyzer to erroneously suck the separating material thereinto. In addition, the separating material having an increased strength can show a high bonding strength to a wall surface so that the cell-containing component can be prevented from leaking into the serum or plasma component through an interface between the separating material and the wall surface.
Examples of the reinforcing material usable in the present invention include polystyrenes, polyurethanes, acrylic resins, polyolefins and silicone resins. Among these reinforcing materials, preferred are polystyrenes. Further, as the reinforcing material, there may also be used the molded articles as a cured product of the moisture curing component contained in the separating material.
The specific gravity of the reinforcing material is preferably from 1.03 to 1.09, more preferably from 1.03 to 1.07, still more preferably from 1.035 to 1.055, and especially preferably is similar to that of the separating material, in order to place the reinforcing material at the position intermediate between the cell-containing component and the serum or plasma component. The amount of the reinforcing material added to the separating material is preferably from 2 to 900 parts by mass on the basis of 100 parts by mass of the curing component. When the amount of the reinforcing material added is 2 parts by mass or more on the basis of 100 parts by mass of the curing component, the separating material can be enhanced in strength and ensure a good bonding property to the wall surface. When the amount of the reinforcing material added is 900 parts by mass or less, the separating material is free from significant deterioration of its fluidity, has a sufficient function of separating the cell-containing component and the serum or the like from each other, and ensures a good bonding property to a wall surface of the tube. From the above viewpoints, the amount of the reinforcing material added to the separating material is more preferably from 5 to 250 parts by mass and still more preferably from 10 to 100 parts by mass on the basis of 100 parts by mass of the curing component.
The reinforcing material may be added to the separating material either in the form of a mixture with the curing component or separately from the curing component. More specifically, the reinforcing material in the form of a powder is preferably mixed as a filler in the curing component, followed by enclosing the resulting mixture in a capsule, a container, etc., as described below. The reinforcing material in the form of beads may be mixed in the curing component, may be enclosed together with the curing component in a capsule, a container, etc., or may be disposed outside of a blood isolating material such as a capsule and a container. Also, the reinforcing material in the form of a molded article may be enclosed together with the curing component in a capsule, a container, etc., or may be disposed outside of a blood isolating material such as a capsule and a container (refer to FIGS. 9-1 to 9-3 and FIGS. 10-1 to 10-3). In any of the above configurations, the reinforcing material is at least partially incorporated in a cured product of the curing component upon the curing to thereby enhance a strength of the resulting separating material.

Next, the method in which a molded article is used as the reinforcing material and disposed in the collection tube is explained. For example, as shown in FIG. 9-1, the moisture curing component 4 and the molded article 51 are disposed inside the blood isolating material 5 disposed in the collection tube. Even when blood is collected in the collection tube, curing of the moisture curing component does not occur since the blood isolating material 5 is disposed above the moisture curing component (FIG. 9-2). When being subjected to centrifugal separation, the blood isolating material 5 is broken or displaced by the molding article 51 to allow the moisture curing component 4 and the blood to come into contact with each other, so that curing of the moisture curing component is initiated. As shown in FIGS. 9-1 to 9-3, the molded article may be disposed together with the moisture curing component 4 inside of the blood isolating material 5. Alternatively, as shown in FIGS. 10-1 to 10-3, the molded article may be disposed outside of the blood isolating material 5, for example, above the blood isolating material 5. In the present invention, either a single molded article or a plurality of molded articles may be used. In addition, the molded article may have various shapes such as a cylindrical shape, a disk shape, a spherical shape and a rectangular parallelopiped shape. The material of the molded article may be the same as or different from the separating material or the curing component contained in the separating material. In addition, as the material of the moulded article, there may also be used the same material as the solid blood isolating material. Further, a high-specific gravity solid, etc., may be disposed to cause breakage or displacement of the blood isolating material.

The specific gravity of the molded article is preferably similar to the specific gravity of the curing component contained in the separating material. More specifically, the specific gravity of the moulded article is preferably from 1.03 to 1.09, more preferably from 1.03 to 1.07 and still more preferably from 1.035 to 1.055. As shown in FIGS. 9-3 to 10-3, after being subjected to the centrifugal separation, the moisture curing component 4 is cured after or while the moisture curing component is disposed at the position between the serum or the like 7 and the cell-containing component 8, so that the cured moisture curing component can cooperate with the molded articles 51 to prevent the upper and lower blood components from being mixed with each other.
Meanwhile, the molded article as the reinforcing material may also be disposed in the collection tube shown in each of FIGS. 1-1 to 1-3 through FIGS. 8-1 to 8-3. When using the molded article having a specific gravity similar to that of the curing component contained in the separating material as described above, the amount of the curing component used can be reduced. In addition, since erroneous absorption of the curing component upon pipetting can be prevented even when being present in an uncured state, the curing time of the curing component usable therein can be prolonged. Further, when being subjected to centrifugal separation after collection of blood, occurrence of such a risk that the blood is entrained in the curing component can be reduced.

In addition, the separating material used in the present invention may also contain a tackifier in order to enhance a bonding property of the separating material to a wall of a test tube. As the tackifier, there may be used, for example, silane coupling agents. Specific examples of the silane coupling agents include aminopropyl trimethoxysilane and glycidyl triethoxysilane.

Next, the materials used in the respective methods described above are explained in detail below.

First, the moisture curing component as used in the above (1) means a component capable of undergoing a curing reaction in the presence of water, Examples of the moisture curing component include those resins or compounds which contain at least one hydrolyzable reactive group or at least one functional group capable of initiating a reaction thereof by the action of water in a molecule thereof, and can initiate a curing reaction thereof by the action of water in ambient air, etc. In the present invention, the moisture curing component is not particularly limited, and any moisture curing component having the above specific gravity may be used as long as it can initiate a curing reaction thereof by contacting with water in blood. Specific examples of the moisture curing component include a reactive silicone-based compound, an α-cyanoacrylate-based compound, a one-part liquid moisture curing polyurethane resin, a moisture curing epoxy resin composition and a moisture curing polysulfide resin composition. Among these moisture curing components, the reactive silicone-based compound, the α-cyanoacrylate-based compound and the one-part liquid moisture curing polyurethane resin are preferably used in view of a high curing rate and a less adverse influence on blood tests, and the reactive silicone-based compound is more preferably used in view of a high bonding property to a wet surface and less occurrence of peel-off from a wall surface upon temperature change owing to a good elasticity thereof.

Examples of the suitable reactive silicone-based compound include moisture curing silicone resins having a polysiloxane structure in a main chain thereof and containing a reactive group capable of initiating a curing reaction by reacting with water at a terminal end thereof, and modified silicone-based resins in the form of a polymer having, in addition to the polysiloxane structure, a polyether, polyester or poly(meth)acrylic acid ester structure, etc., in a main chain thereof, which contain at least one reactive curing group per a molecule of the polymer. The reactive curing group means a functional group having such a structure capable of forming a silanol group by reacting with water. Examples of the above silicone-based resins containing such a reactive curing group include dealcoholation type silicone resins, carboxylic acid-desorbing (decarboxylation) type silicone resins such as acetic acid-desorbing (deacetylation) type silicone resins, deoximation type silicone resins, deamidation type silicone resins, deamination type silicones resins and deacetonation type silicone resins, depending upon the kind of group to be desorbed therefrom by the reaction. Among these silicone-based resins containing the reactive curing group, preferred are the dealcoholation type modified silicone resins such as "KANEKA SILYL SAX220" and "KANEKA SILYL SAT400" both available from Kaneka Corp.

Next, typical examples of the suitable α-cyanoacrylate-based compound include those compounds represented by the following general formula (I):

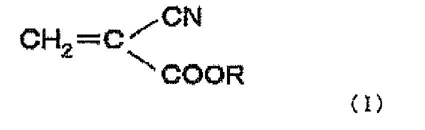

Examples of the group R in the general formula (I) include alkyl groups such as a methyl group, an ethyl group, an n-propyl group, an n-butyl group, an isobutyl group and an n-pentyl group; alkenyl groups; a cyclohexyl group; aryl groups; and alkoxyalkyl groups.
In general, the α-cyanoacrylate-based compound rapidly initiates anion polymerization in the presence of water as a curing catalyst, so that the curing reaction of the α-cyanoacrylate-based compound proceeds at a very high rate. Therefore, in such a case, the above blood isolating material including the capsule is preferably used.
In addition, when the group R in the general formula (I) is a low molecular weight alkyl group such as a methyl group and an ethyl group, the α-cyanoacrylate-based compound is a low-viscosity liquid and therefore may be difficult to handle as a separating material. For this reason, the α-cyanoacrylate-based compound is preferably adjusted in curing rate and viscosity thereof in order to improve a handling property thereof as a separating material. In order to suitably adjust the curing rate and viscosity of the α-cyanoacrylate-based compound as the moisture curing component, there may be used a method of compounding a large amount of the other resin or compound which is inert to the moisture curing reaction of the α-cyanoacrylate-based compound, or a method of using the α-cyanoacrylate-based compound of the general formula (I) in which the group R is a long-chain straight alkyl group or branched alkyl group having 8 or more carbon atoms to enhance a viscosity of the compound or reduce a curing rate thereof. Specific examples of the other resin include poly(meth)acrylic acid esters, polyesters and polyacrylonitrile, Examples of the long-chain alkyl group include an n-octyl group, a lauryl group, a stearyl group and an isostearyl group.

Examples of the one-part liquid moisture curing polyurethane resin include polyisocyanate urethane prepolymers having a plurality of isocyanate groups at a terminal end thereof which are obtained by reacting a polyisocyanate with a polyol, a polyether polyol, a polyhydric phenol or the like. The isocyanate groups are reacted with water while generating a carbon dioxide gas to thereby allow the urethane prepolymers to undergo a crosslinking reaction. Specific examples of the polyisocyanate include aliphatic polyisocyanates such as hexamethylene diisocyanate; alicyclic polyisocyanates such as dicyclohexylmethane diisocyanate and isophorone diisocyanate; and aromatic polyisocyanates such as tolylene diisocyanate, diphenylmethane diisocyanate, p-phenylene diisocyanate, naphthylene diisocyanate and xylylene diisocyanate.
Specific examples of the polyol include ethylene glycol, propylene glycol, 1,3-butylene glycol, 1,4-butylene glycol, neopentyl glycol, hydrogenated bisphenol A, hydrogenated bisphenol F, polytetramethylene glycol, polyester diols, trimethylol propane, 1,2,4-butanetriol, 1,2,6-hexanotriol, glycerol and pentaerythritol. Specific examples of the polyhydric phenol include bisphenol A and bisphenol F. Specific examples of the polyether polyol include adducts of the above polyol or polyhydric phenol with an alkyleneoxide such as ethyleneoxide and propyleneoxide.

The one-part liquid moisture curing polyurethane resin usable in the present invention may be produced by an ordinary synthesis method in which the above polyisocyanate and polyol, etc., are compounded with each other in such an amount that a ratio of an NCO group to an OH group therein is usually in the range of from 1.5 to 5.0 and preferably from 1.7 to 3.0. The content of isocyanate groups in the one-part liquid moisture curing polyurethane resin is usually from 0.5 to 20% by mass, preferably from 1 to 10% by mass and more preferably from 2 to 8% by mass. When the isocyanate group content is 0.5% by mass or more, the effect of enhancing a curing rate of the one-part liquid moisture curing polyurethane resin can be sufficiently attained, so that the serum or plasma and the cell-containing component can be sufficiently separated from each other. On the other hand, when the isocyanate group content is 20% by mass or less, the curing rate of the one-part liquid moisture curing polyurethane resin can be readily controlled without becoming excessively high.

The separating material used in the present invention may also contain an ordinary curing catalyst for curing the moisture curing component, if required. The content of the curing catalyst in the separating material is usually in the range of from 0.01 to 20 parts by mass on the basis of 100 parts by mass of the moisture curing component. When the content of the curing catalyst in the separating material is 0.01 part by mass or more, a sufficient curing rate of the moisture curing component can be attained, so that the serum or plasma and the cell-containing component can be sufficiently separated from each other. On the other hand, when the content of the curing catalyst in the separating material is 20 parts by mass or less, the curing rate of the moisture curing component can be kept at an adequate lever without becoming excessively high.

For example, when using the reactive silicone-based compound as the moisture curing component, the separating material used in the present invention may contain a curing catalyst such as organic tin compounds, metal complexes and organic phosphorus oxides, if required. Specific examples of the curing catalyst include tin compounds such as dibutyl tin dilaurate, dibutyl tin phthalate and stannous octylate; titanate compounds, e.g., titanium alkoxides such as tetrabutyl titanate and tetraisopropyl titanate, titanium chelates such as "ORGATIX TC-750" and "ORGATIX T-2970" both available from Matsumoto Fine Chemical Co., Ltd., titanium acylates, and triethanol amine titanate; organic zirconium compounds such as zirconium alkoxides, zirconium acylates and zirconium chelates; carboxylic acid metal salts such as lead octylate, lead naphthenate, nickel naphthenate and cobalt naphthenate; metal acetylacetate complexes such as aluminium acetylacetate complex and vanadium acetylacetonate complex; and amine salts such as dibutyl amine-2-ethyl hexoate. Among these curing catalysts, preferred are tin compounds and titanate compounds, and more preferred are titanate compounds. Further, among these titanate compounds, still more preferred are titanium chelates. However, these curing catalysts may give adverse influence on the results of blood tests depending upon some test items. Therefore, in such a case, it is preferable to use none of the curing catalysts in the separating material.
In addition, when using the titanium compound as the curing catalyst in the separating material, the moisture curing component tends to be colored yellow. In this case, the color of a resin as the moisture curing component is changed to white color or light yellow color when being cured. Therefore, by observing the change in color of the resin, it is possible to suitably recognize an extent of curing of the moisture curing component from outside of the collection tube.
The content of the curing catalyst in the separating material is preferably from 0.01 to 10 parts by mass, more preferably from 0.1 to 5 parts by mass and still more preferably from 0.2 to 3 parts by mass on the basis of 100 parts by mass of the reactive silicone-based compound in order to attain a sufficient curing rate of the moisture curing component. When the content of the curing catalyst in the separating material is 0.01 part by mass or more, a sufficient effect of enhancing a curing rate of the moisture curing component can be attained. When the content of the curing catalyst in the separating material is 10 part by mass or less, an excessive increase in curing rate of the moisture curing component can be prevented, and the separating material can exhibit a sufficient storage stability.

When using the one-part liquid moisture curing polyurethane resin as the moisture curing component, the separating material used in the present invention may also contain, if required, a curing catalyst, e.g., an organic metal catalyst such as tin compounds such as dibutyl tin dilaurylate and titanium compounds, and tertiary amine compounds such as triethyl amine and triethylene diamine.
However, these curing catalysts might give adverse influence on the results of blood tests depending upon some test items. Therefore, in such a case, it is preferable to use none of the curing catalysts in the separating material. The amount of the curing catalyst compounded in the separating material is preferably from 0.01 to 10 parts by mass on the basis of 100 parts by mass of the one-part liquid moisture curing polyurethane resin in order to attain a sufficient curing rate of the moisture curing component. When the content of the curing catalyst compounded is 0.01 part by mass or more, a sufficient effect of enhancing a curing rate of the one-part liquid moisture curing polyurethane resin can be attained. When the content of the curing catalyst compounded is 10 parts by mass or less, an excessive increase in curing rate of the one-part liquid moisture curing polyurethane resin can be prevented, and the separating material can exhibit a sufficient storage stability.

The separating material used in the present invention may also contain, in addition to the moisture curing component such as the above moisture curing resin or compound, the other resin or compound having no reactivity by itself, and/or the other curing resin or compound of a different curing type such as those of a heat-curing type and an electron radiation curing type, if required.

Next, the two-part liquid mixing type component used in the above (2) is generally in the form of a two-part liquid mixing type resin. Examples of the two-part liquid mixing type resin suitably used in the present invention include those curing resins ordinarily used in paints or molding materials such as various urethane curing resins, acid-epoxy curing resins and epoxy-amine curing resins. Among these curing resins, preferred are urethane curing resins.

Examples or the materials of the surface-treated beads used in the above (3) include organic materials, e.g., polyolefins such as polyethylene and polypropylene, polystyrenes, acrylic resins and polyesters such as polyethylene terephthalate, and inorganic materials such as silica, glass and ceramics. These materials may be used alone or in combination of plural kinds thereof. The specific gravity of these materials is preferably controlled to the range of from 1.03 to 1.09, more preferably from 1.03 to 1.07 and especially preferably from 1.035 to 1.055.
The specific gravity of the beads may be controlled by the method of combining the materials which are different in specific gravity from each other, the method of forming hollow beads, etc. The surface treating method of the beads may be the same as described above.

The collection tube usable in the method of the present invention is not particularly limited, and any conventionally known collection tubes may be used as such. The material of the collection tube may also be the same material as used conventionally. Examples of the material of the collection tube include glass, and plastic materials such as polyesters, polyethylene, polypropylene and polymethyl methacrylate. Examples of commercially available products of the collection tube include "VENOJECT (registered trademark) II" available from Terumo Corp., etc.
The inner wall surface of the collection tube may be subjected to surface treatments to facilitate bonding of the separating material thereto upon curing thereof. For example, the inner wall surface of the collection tube may be subjected to an acid or alkali treatment, a silane coupling treatment, a light irradiation treatment, an ozone treatment or the like. These surface treatments enables introduction of a functional group to a wall surface of the collection tube to thereby obtain the effect of facilitating the reaction between the wall surface and the separating material.

Also, the collection tube may be charged with additives such as blood coagulation accelerators for promoting coagulation of blood, and blood anti-coagulation agents for suppressing coagulation of blood according to kinds of blood inspection or examination items. Examples of the blood coagulation accelerators include protamine sulfate, thrombin, silica sand, crystalline silica powder, diatomaceous earth, glass powder, kaolin and bentonite. Examples of the blood anti-coagulation agents include heparin and EDTA (ethylenediaminetetraacetic acid).
Meanwhile, in the present invention, when it is intended to obtain a serum as a supernatant by the centrifugal separation, the above coagulation accelerator may be added to blood in the collection tube, whereas when it is intended to obtain a plasma, the above anti-coagulation agent may be added to blood in the collection tube.
The amount of the respective additives added may vary depending upon the kinds of additives used, and is usually in the range of from 0.3 to 10.0 mg per 10 mL of the blood collected in the collection tube. When the amount of the additive added is 0.3 mg or larger, the respective additives can suitably exhibit effects thereof. When the amount of the additive added is 10.0 mg or smaller, there occur no significant problems concerning hemolysis.

The hardness of the separating material used in the present invention after being cured is preferably controlled such that the resulting cured separating material has a strength capable of withstanding breakage thereof even when contacting with a tip end of a pipette upon dispensing the respective blood components, or a strength capable of avoiding occurrence of breakage thereof owing to vibration upon transportation or handling.

In the method of separating serum or plasma according to the present invention, blood is collected in the collection tube in which the serum or plasma separating material, is previously disposed, and then the contents of the collection tube are subjected to centrifugal separation. The centrifugal separation method may be the same as used conventionally. For example, the centrifugal separation procedure may be carried out for about 10 min while applying a centrifugal force of about 1200 G to the contents of the collection tube to thereby separate the serum or plasma and the well-containing component from each other.
More specifically, since the specific gravity of the serum or plasma separating material used in the present invention is usually intermediate between those of the serum or the like and the cell-containing component, the separating material is disposed and cured at an intermediate position between the serum or the like and the cell-containing component in the collection tube while keeping both the blood components in a separated state. Therefore, it is possible to separate the serum or the like and the cell-containing component from each other merely by being subjected to the centrifugal separation, and prevent these separated blood components from being mixed again with each other. Accordingly, even when the blood specimen is transported after separation of the respective blood components from a hospital to a blood inspection or examination center, etc., the serum or the like and the cell-containing component can be inhibited from being re-mixing with each other.

### EXAMPLES

The present invention will be described in more detail by referring to the following Examples. However, it should be noted that these examples are only illustrative and not intended to limit the invention thereto.

### EXAMPLE 1

A stored horse blood (available from Kohjin Bio Co., Ltd.; a mixture containing a horse blood and an Alsever's solution at a mixing ratio of 1:1) was prepared. Further, 2 mL of a moisture curing silicone resin "TSE397" (one-component condensed type (dealcoholation type) silicone resin; specific gravity: 1.04; viscosity: 50 Pa·s) available from Momentive Performance Materials Japan Inc., as a moisture curing component, were filled in a low-density polyethylene tube (LDPE tube; outer diameter: 11 mm; thickness: 0.4 mm; length: 20 mm), and each of upper and lower open ends of the tube was closed by a lid formed of a Parafilm ("PM-992" available from Pechiney Plastic Packaging, Inc.) to form a capsule. The thus formed capsule enclosing the moisture curing component was used.

A collection tube (vacuum blood collection tube filled with a curing accelerator; available from Terumo Corp.) was opened with a lid being off, and the capsule enclosing the moisture curing silicone resin was placed in the collection tube. On the capsule was disposed a high-specific gravity solid (shape: spherical shape; diameter: 6 mm; material: glass; specific gravity: 2.5). Then, 8 mL of the stored horse blood were charged into the collection tube, and the open end of the collection tube was closed with a lid formed of a plastic film, and the thus filled collection tube was allowed to stand for 3 h and then subjected to centrifugal separation to separate the blood into serum or the like and a cell-containing component. The centrifugal separation was carried out at 3000 rpm (1200 G) for 10 min. Thereafter, the thus separated blood components in the collection tube were allowed to stand for 3 h, and the plasma component was separated therefrom by decantation. Then, a cured product of the moisture curing component was pushed with a wood bar having a length of 10 cm and a diameter of 2 mm. As a result, it was confirmed that the moisture curing component was cured sufficiently. Although the plasma component was separated from the blood, slight hemolysis was observed.

### COMPARATIVE EXAMPLE 1

The same centrifugal separation procedure as in Example 1 was repeated except that the moisture curing silicone resin was not enclosed in the capsule. As a result, it was confirmed that the moisture curing silicone resin was already cured before subjected to the centrifugal separation, and the blood was not subjected to centrifugal separation.

### EXAMPLE 2

A stored horse blood (available from Kohjin Bio Co., Ltd.; a mixture containing a horse blood and an Alsever's solution at a mixing ratio of 1:1) was prepared. A moisture curing silicone resin "TSE397" (one-component condensed type (dealcoholation type) silicone resin; specific gravity: 1.04; viscosity: 50 Pa·s) available from Momentive Performance Materials Japan Inc., as a reinforcing material, was cured to form a cylindrical molded article (diameter: 11 mm; height: 6 mm; weight: 0.6 g).

A collection, tube (vacuum blood collection tube filled with a curing accelerator; available from Terumo Corp.) was opened with a lid being off, and then 0.9 mL of the moisture curing silicone resin "TSE397" as a moisture curing component was filled in a bottom of the collection tube. The moisture curing component thus filled in the collection tube was covered with a lid formed of a polyethylene film, and the molded article was placed on the polyethylene film. Then, 8 mL of the stored horse blood was charged into the collection tube, and the open end of the collection tube was closed with a lid formed of a plastic film. The thus filled collection tube was then subjected to centrifugal separation to separate the blood into serum or the like and a cell-containing component. The centrifugal separation was carried out at 3000 rpm (1200 G) for 10 min. Thereafter, the collection tube including the thus separated blood components was allowed to stand for 3 h, and the plasma component was separated therefrom by decantation. Then, a cured product of the moisture curing component was pushed with a wood bar having a length of 10 cm and a diameter of 2 mm. As a result, it was confirmed that the moisture curing component was cured sufficiently. Although the plasma component was separated from the blood, slight hemolysis was observed.

### EXAMPLE 3

A stored horse blood (available from Kohjin Bio Co., Ltd.) was prepared, and 94% by mass of a modified silicone ("SAT400" available from Kaneka Corp.; viscosity: 24 Pa·s) as a moisture curing component were mixed with 6.0% by mass of calcium carbonate (available from Wako Pure Chemical Industries, Ltd.) as a specific gravity modifier to adjust a specific gravity of the resulting mixture to 1.05. Then, 0.5 part by mass of a titanium-based curing catalyst ("TC-750" available from Matsumoto Fine Chemical Co., Ltd.) was added to 100 parts by mass of the above mixture (moisture curing component) to prepare a curing composition. Then, a polypropylene container (a round bottom tube having a diameter of 1 cm and a length of 2 cm) was filled with 1.5 mL of the curing composition, and an aluminum film (available from Nippon Foil Manufacturing Co., Ltd.; thickness: 0.02 mm) as a blood isolating material was heat-bonded to an open end of the container to close the container with the lid, thereby obtaining a capsule.

A collection tube (vacuum blood collection tube filled with a curing accelerators; available from Terumo Corp.) was opened with a lid being off, and the capsule was placed in the collection tube, and then glass beads (diameter: 6 mm) were disposed on the capsule. Then, 8 mL of the stored horse blood were charged into the collection tube, and the open end of the collection tube was closed with a lid for recapping, and the thus filled collection tube was subjected to centrifugal separation. The centrifugal separation was carried out at 3000 rpm (1200 G) for 10 min. The capsule was broken upon the centrifugal separation, so that resin filled therein was discharged out of the capsule and disposed between the plasma component and the cell-containing component. As a result, it was confirmed that the plasma component was sufficiently separated from the blood, and any cell-containing component was not included in the plasma component.

### EXAMPLE 4

The same separating procedure as in Example 3 was repeated except that the following components were filled in the polypropylene container, and the thus filled container was closed with a lid for recapping and, after the elapse of 1 day, subjected to centrifugal separation.

That is, 86.35% by mass of a modified silicone ("EST280" available from Kaneka Corp.; viscosity. 7 Pa·s) as a moisture curing component were mixed with 13.65% by mass of "Bentone 38" (available from Elementis Specialties Inc.) as a specific gravity modifier to adjust a specific gravity of the resulting mixture to 1.05. Then, 0.5 part by mass of a titanium-based curing catalyst ("TC-750" available from Matsumoto Fine Chemical Co., Ltd.) was added to 100 parts by mass of the above mixture (moisture curing component) to prepare a component to be filled in the above polypropylene container.

The capsule was broken upon the centrifugal separation, so that the resin filled therein was discharged out of the capsule and disposed between the plasma component and the cell-containing component. As a result, it was confirmed that the plasma component was sufficiently separated from the blood, and any cell-containing component was not included in the plasma component.

### EXAMPLE 5

The same separating procedure as in Example 3 was repeated except that in Example 3, the following components were filled in the polypropylene container, and the thus filled container was closed with a lid for recapping ("Venoject II Recap" available from Terumo Corp.) and, after the elapse of 1 day, subjected to centrifugal separation.

That is, 91% by mass of a modified silicone ("SAT400" available from Kaneka Corp.; viscosity: 24 Pa·s) as a moisture curing component were mixed with 9% by mass of silica particles ("OX50" available from Nippon Aerosil Co., Ltd.; particle size: 40 mm) as a specific gravity modifier to adjust a specific gravity of the resulting mixture to 1.05. Then, 0.5 part by mass of a titanium-based curing catalyst ("TC-750" available from Matsumoto Fine Chemical Co., Ltd.) was added to 100 parts by mass of the above mixture (moisture curing component) to prepare a component to be filled in the above polypropylene container.

The capsule was broken upon the centrifugal separation, so that the moisture curing component filled therein was discharged out of the capsule and disposed between the plasma component and the cell-containing component. As a result, it was confirmed that the plasma component was sufficiently separated from the blood, and any cell-containing component was not included in the plasma component.

### EXAMPLE 6

A stored horse blood (available from Kohjin Bio Co., Ltd.) was prepared, and 91% by mass of a modified silicone ("SAT400" available from Kaneka Corp.; viscosity: 24 Pa·s) as a moisture curing component were mixed with 9% by mass of silica particles ("OX50" available from Nippon Aerosil Co., Ltd.; particle size: 40 nm) as a specific gravity modifier to adjust a specific gravity of the resulting mixture to 1.05. Then, 0.5 part by mass of a titanium-based curing catalyst ("TC-750" available from Matsumoto Fine Chemical Co., Ltd.) and 30 parts by mass of polystyrene beads (a spherical shape having a diameter of 0.3 mm; available from Hitachi Chemical Co., Ltd.) as a reinforcing material were added to 100 parts by mass of the above mixture (moisture curing component) to prepare a curing composition. Then, a polypropylene container, (a round bottom tube having a diameter of 1 cm and a length of 2 cm) was filled with 1.5 mL of the curing composition, and an aluminum film (available from Nippon Foil Manufacturing Co., Ltd.; thickness: 0.02 mm) as a blood isolating material was heat-bonded to an open end of the container to close the container with the lid, thereby obtaining a capsule.

A collection tube (vacuum blood collection tube filled with a curing accelerator; available from Terumo Corp.) was opened with a lid being off, and the capsule was placed in the collection tube, and then glass beads (diameter: 6 mm; specific gravity: 2.5) as a high-specific gravity solid were disposed on the capsule. Then, 8 mL of the stored horse blood were charged into the collection tube, and the open end of the collection tube was closed with a lid for recapping ("Venoject II Recap" available from Terumo Corp.), and, after the elapse of 1 day, the thus filled collection tube was subjected to centrifugal separation. The centrifugal separation was carried out at 3000 rpm (1200 G) for 10 min. The capsule was broken upon the centrifugal separation, so that the moisture curing component filled therein, was discharged out of the capsule and disposed between the plasma component and the cell-containing component. As a result, it was confirmed that the plasma component was sufficiently separated from the blood, and any cell-containing component was not included in the plasma component.

### EXAMPLE 7

A stored horse blood (available from Kohjin Bio Co., Ltd.) was prepared. A silicone resin "TSE397" (one-component condensed type (dealcoholation type) silicone resin; specific gravity: 1.04; viscosity: 50 Pa·s) available from Momentive Performance Materials Japan Inc., was used as a moisture curing component. In addition, the moisture curing silicone resin "TSE397" (one-component condensed type (dealcoholation type) silicone resin; specific gravity: 1.04; viscosity: 50 Pa·s) available from Momentive Performance Materials Japan Inc., as a reinforcing material was cured to form a cylindrical molded article (diameter: 11 mm; height: 6 mm; weight: 0.6 g).

A collection tube (vacuum blood collection tube filled with a curing accelerator; available from Terumo Corp.) was opened with a lid being off, and then the molded article formed above as the reinforcing material was placed and disposed within the collection tube. Further, the collection tube was charged with 1 mL of the above moisture curing component and then with 8 mL of the stored horse blood, and the open end of the collection tube was closed with a lid for recapping ("Venoject II Recap" available from Terumo Corp.). Then, the thus filled collection tube was subjected to centrifugal separation. The centrifugal separation was carried out at 3000 rpm (1200 G) for 10 min. As a result, it was confirmed that although the plasma component was sufficiently separated from the blood, very slight hemolysis was observed.

### EXAMPLE 8

A stored horse blood (available from Kohjin Bio Co., Ltd.) was prepared. Further, 93.75% by mass of a modified silicone ("SAX220" available from Kaneka Cop.; viscosity: 46 Pa·s) as a moisture curing component were mixed with 6.25% by mass of calcium carbonate (available from Wako Pure Chemical Industries, Ltd.) as a specific gravity modifier to adjust a specific gravity of the resulting mixture to 1.05. Then, 1 part by mass of a titanium-based curing catalyst ("TC-750" available from Matsumoto Fine Chemical Co., Ltd.) was added to 100 parts by mass of the above mixture (moisture curing component), and further a polystyrene cylindrical molded article (diameter: 9 mm; height: 6 mm; specific gravity: 1.05; weight: 0.4 g) as a reinforcing material was added to the resulting mixture to thereby prepare a separating material.

A collection tube (vacuum blood collection tube filled with a curing accelerator; available from Terumo Corp.) was opened with a lid being off, and the polystyrene cylindrical molded article was placed in the collection tube. In addition, the collection tube was charged with 1.3 mL of the above mixture containing the moisture curing component and the curing catalyst. Then, 8 mL of the stored horse blood were further charged into the collection tube, and the open end of the collection tube was closed with a lid for recapping ("Venoject II Recap" available from Terumo Corp.), followed by subjecting the thus filled collection tube to centrifugal separation. The centrifugal separation was carried out at 3000 rpm (1200 G) for 10 min. As a result, it was confirmed that the plasma component was sufficiently separated from the blood, and any cell-containing component was not included in the plasma component.

### EXAMPLE 9

The procedure was carried out in the same manner as in Example 8 using the same blood, moisture curing component, curing catalyst and reinforcing material as those used in Example 8 except that the separation procedure was changed as follows. That is, the same separating procedure as in Example 8 was repeated except that a collection tube (vacuum blood collection tube filled with a curing accelerator; available from Terumo Corp.) was opened with a lid being off, and 1.3 mL of the mixture containing the moisture curing component and the curing catalyst were charged into the collection tube, and then the polystyrene cylindrical molded article (diameter: 9 mm; height: 6 mm; specific gravity: 1.05; weight: 0.4 g) as a reinforcing material was disposed on the mixture. As a result, it was confirmed that the plasma component was sufficiently separated from the blood, and any cell-containing component was not included in the plasma component.

### INDUSTRIAL APPLICABILITY

In accordance with the method of the present invention, even when it takes a long time from collection of blood to inspection or examination thereof, the blood can be surely separated into serum or plasma and a cell-containing component by centrifugal separation, and after the centrifugal separation, the serum or plasma and the cell-containing component thus separated from each other can be stored in a separated state with a good storage stability for a long period of time, and further the separation of the serum or plasma can be carried out with an excellent stability upon freezing or thawing as well as upon handling of the sample. That is, the serum or plasma and the well-containing component are prevented from being mixed with each other even after the elapse of time, so that a blood test can be carried out with a high accuracy.
In addition, the separating material used in the method of the present invention can be cured without using an ultraviolet ray. Therefore, the blood test can be carried out without taking into consideration adverse influence of the ultraviolet ray, and further it is possible to conduct a sterilization procedure by irradiation with ultraviolet ray or γ-ray.

## Claims

1. A method of separating serum or plasma comprising the steps of disposing a serum or plasma separating material in a blood collection tube; collecting blood in the collection tube; and subjecting the collection tube filled with the blood to centrifugal separation, wherein the serum or plasma separating material comprises a curing component, and curing of the curing component is initiated by the centrifugal separation.

2. The method of separating serum or plasma according to claim 1, wherein the serum or plasma separating material further comprises a blood isolating material.

3. The method of separating serum or plasma according to claim 2, wherein the blood isolating material is in the form of a capsule in which the curing component is enclosed.

4. The method of separating serum or plasma according to any one of claims 1 to 3, wherein the serum or plasma separating material further comprises a reinforcing material.

5. The method of separating serum or plasma according to claim 4, wherein the reinforcing material is in the form of a molded article.

6. The method of separating serum or plasma according to any one of claims 1 to 5, wherein the curing component has a specific gravity of from 1.03 to 1.09.

7. The method of separating serum or plasma according to any one of claims 1 to 6, wherein the curing component is a moisture curing component, and the moisture curing component is brought into contact with the blood by the centrifugal separation to thereby initiate curing of the moisture curing component.

8. The method of separating serum or plasma according to any one of claims 1 to 6, wherein the curing component is a two-part liquid curing type component, and two liquids in the two-part liquid curing type component are brought into contact with each other by the centrifugal separation to thereby initiate curing of the two-part liquid curing type component.

9. The method of separating serum or plasma according to any one of claims 1 to 6, wherein the curing component is in the form of surface-treated resin beads, and the surface-treated resin beads are brought into contact with each other by the centrifugal separation to thereby initiate curing of the surface-treated resin beads.

10. The method of separating serum or plasma according to claim 7, wherein the moisture curing component is at least one material selected from the group consisting of a reactive silicone-based compound, an α-cyanoncrylate-based compound and a one-part liquid moisture curing polyurethane resin.
